# EUROPÄISCHE PATENTSCHRIFT

(11) **EP 2 336 418 B1**
(45) Veröffentlichungstag und Bekanntmachung des Hinweises auf die Patenterteilung: **28.08.2013**
(21) Anmeldenummer: 10193296.0
(22) Anmeldetag: 01.12.2010
(51) Int. Cl.: D06F 58/20, C11D 17/04, A47L 15/42, A61L 9/012, A61L 9/12

(54) **Riechstoffhaltige Hohlkörper bestehend aus einer Polymermatrix**
Cavity containing odorous substances consisting of a polymer matrix
Corps creux contenant du parfum constitué d'une matrice polymère

(30) Priorität: 02.12.2009 DE 102009047411
(43) Veröffentlichungstag der Anmeldung: 22.06.2011
(73) Patentinhaber: Symrise AG, 37603 Holzminden (DE)
(72) Erfinder: Wiedemann, Jörn, 37603, Holzminden (DE); Hielscher, Thorsten, 37671, Höxter (DE); Harzke, Falk, 37619, Bodenwerder (DE)
(74) Vertreter: Eisenführ, Speiser & Partner

(56) Entgegenhaltungen:
- US-A- 3 676 199
- US-A1- 2006 010 933
- US-B1- 6 174 577
- Amazon.de: "Duftbälle", , 25. März 2007 (2007-03-25), XP002625429, Gefunden im Internet: URL:http://www.amazon.de/Sport-Thieme-Duft b%C3%A4lle/dp/B001C37T12/ref=sr_1_2?ie=UTF 8&qid=1298964643&sr=8-2 [gefunden am 2011-03-01]

## Beschreibung

Die vorliegende Erfindung betrifft einen Hohlkörper umfassend oder bestehend aus einer elastomeren Polymermatrix, in der ein oder mehrere Riechstoffe und gegebenenfalls weitere Stoffe eingelagert sind, und welcher unter den vorherrschenden Bedingungen eines Standard-Wäschetrockenvorganges in einem Wäschetrockner und/oder eines Standard-Spülvorganges in einem Geschirrspüler den oder die Riechstoffe freisetzt und auf die Wäsche aufbringt bzw. die Luft in dem Geschirrspüler beduftet, wobei der Körper vorzugsweise seine strukturelle Integrität behält, d.h. keine optisch sichtbaren Schädigungen erleidet.

Die vorliegende Erfindung betrifft auch die Verwendung eines Hohlkörpers umfassend oder bestehend aus einer elastomeren Polymermatrix, in der ein oder mehrere Riechstoffe und gegebenenfalls weitere Stoffe eingelagert sind, zum Beduften von Wäsche während eines Standard-Wäschetrockenvorganges in einem Wäschetrockner und/oder zum Beduften der Luft in einem Geschirrspüler während eines Standard-Spülvorganges.

Die vorliegende Erfindung betrifft zudem ein Verfahren zum Herstellen eines derartigen Hohlkörpers, der zum Beduften von Wäsche während eines Wäschetrockenvorganges in einem Wäschetrockner und/oder zum Beduften der Luft in einem Geschirrspüler während eines Standard-Spülvorganges geeignet ist.

Einzelheiten der vorstehend genannten Aspekte der vorliegenden Erfindung sowie weitere Aspekte der Erfindung, insbesondere besonders bevorzugte Ausgestaltungen, ergeben sich aus der folgenden Beschreibung, den Beispielen sowie den beigefügten Patentansprüchen.

In vielen Haushalten und in der Reinigungsindustrie werden heutzutage vermehrt Wäschetrockner eingesetzt. Wäschetrockner besitzen den Vorteil, dass sie die gewaschene, noch nasse, Wäsche in einer schnelleren Zeit trocknen, als es zum Beispiel mit einer Trocknung an der Luft möglich wäre. Zudem kann der Einsatz von Wäschetrocknern im Vergleich zu einer Trocknung im Wohnraum energiesparend sein. Durch den Trocknungsvorgang in einem Trockner ist es jedoch möglich, dass die vorher gewaschene und durch ein entsprechendes Waschmittel mit einem angenehmen Duft versehene Wäsche diesen angenehmen Duft wieder verliert. Bei den Konsumenten besteht jedoch das Bedürfnis, auch nach einem Trockenvorgang eine angenehm duftende Wäsche zu erhalten.

Ferner ist in vielen Haushalten ein Geschirrspüler zugegen. Obwohl Geschirrspüler dem Konsumenten die häufig als zeitraubend und unangenehm angesehene Arbeit des Geschirrabspülens mit der Hand abnimmt, ist das Geschirrspülen mit einem Geschirrspüler auch mit Nachteilen besetzt. Viele Konsumenten beklagen, dass durch den Geschirrspülvorgang unangenehme Gerüche entstehen, die spätestens bei Öffnung des Geschirrspüler nach einem Spülvorgang entweichen. Es besteht demnach ein Bedürfnis der Konsumenten, die dem Geschirrspüler entweichende Luft angenehm zu beduften.

In WO 2005/085404 werden riechstoffhaltige Gegenstände offenbart, die ferner eine Weichspülerkomponente und als Trägermaterial z.B. Polyamide enthalten, wobei die Gegenstände für mindestens 10 Trocknungszyklen in einem Wäschetrockner geeignet sind. Dabei erfüllt dort das Trägermaterial die Aufgabe, den Gegenstand widerstandfähig gegenüber der Temperaturbelastung während des Trocknungsvorganges zu machen. Die dort eingesetzten Trägermaterialien besitzen jedoch den Nachteil, dass sie relativ hohe Schmelztemperaturen haben müssen, damit der Gegenstand bei den Temperaturen während des Trocknungsprozesses (ca. 70 bis maximal 100° C) nicht verflüssigt. Um in derartige Trägermaterialien Parfümöl einarbeiten zu können, müssen diese bei Temperaturen, die höher als die Trocknungstemperaturen der Wäschetrockner liegen, geschmolzen werden. Bei den dementsprechend hohen Schmelztemperaturen während des Herstellungsprozesses verdampfen jedoch in erheblichem Maße die einzuarbeitenden Riechstoffe bzw. ein Riechstoffe enthaltendes Parfümöl wird chemisch und/oder geruchlich merklich nachteilig verändert. Besonders wünschenswert sind riechstoffhaltige Gegenstände, welche einen hohen Prozentanteil an Riechstoffen, bezogen auf den eingesetzten Gegenstand, enthalten. Die in WO 2005/085404 beschriebenen Trägermaterialien sind für die Herstellung von riechstoffhaltigen Gegenständen, insbesondere für solche mit einem hohen Prozentanteil an Riechstoffen, ungeeignet.

In WO 2005/118008, US 2005/0267231 und WO 2008/079892 werden Verbindungen und Gegenstände beschrieben, die eine Polymermatrix und eine immobilisierte aktive Flüssigkeit (z.B. ein Parfumöl) umfassen, sowie deren Herstellungsmethoden und Verwendungen. Die Polymermatrix wird als Reaktionsprodukt aus einem Polyamin mit Epoxyverbindungen, Isocyanaten, Anhydriden oder Acrylaten beschrieben. Die offenbarte Herstellungsmethode ermöglicht eine Herstellung ohne eine Behandlung bei erhöhter Temperatur. Die dort offenbarten Ausführungsformen eines Lufterfrischers werden als robust gegenüber Temperaturschwankungen und Feuchtigkeit beschrieben. In WO 2008/079892 ist ferner ausgeführt, dass die dort offenbarten Gegenstände auch als Wäschepflegemittel einsetzbar sind.

Der Nachteil der in WO 2005/118008, US 2005/0267231 und WO 2008/079892 offenbarten Gegenstände liegt darin, dass sich die dort beschriebenen Gegenstände in eigenen Untersuchungen als nicht ausreichend widerstandsfähig gegenüber den heißen, feuchten Bedingungen gezeigt haben, wie sie beispielsweise in einem Wäschetrockner herrschen (Temperaturen regelmäßig im Bereich von 70 bis 100°C). In weiteren eigenen Untersuchungen wurde gefunden, dass beispielweise Vollkugeln mit einem Durchmesser von mehr als 20 mm (entsprechend einem Volumen von etwa 4,2 CM³ oder mehr) aus einer Polymermatrix mit 10 Gew.-% oder mehr eingeschlossenem Parfümöl, bezogen auf das Gesamtgewicht des Gegenstands, bei Anwendung in einem Wäschetrockner und/oder einem Geschirrspüler nicht stabil waren. Bereits bei einem, insbesondere nach drei oder mehr Standard-Trocknungs- oder Spülzyklen (wie unten beschrieben) brachen die dort beschriebenen Gegenstände auseinander, zerbarsten, zerfielen in kleinere Stücke bzw. zeigten bereits mit dem bloßen Auge erkennbare Risse. Bei einer mehrfachen Anwendung verstärkte sich dieser Effekt, je häufiger der Trocknungs- bzw. Spülvorgang wiederholt wurde. Dabei zerfielen die Gegenstände in immer kleinere Stücke.

Bei entsprechenden eigenen Versuchen mit Vollkugeln, die ebenso der Lehre der Patentanmeldungen WO 2005/118008 und US 2005/0267231 oder WO 2008/079892 entsprachen, jedoch einen Durchmesser von 2 cm oder weniger hatten, wurde zwar beobachtet, dass diese in den beschriebenen Behandlungen nicht auseinanderbrachen, zerbarsten und/oder zerfielen, jedoch konnten diese aufgrund ihrer limitierten Größe insgesamt nur mit einer zu geringen Menge an Riechstoffen beladen werden, weshalb solche Gegenstände insbesondere nicht geeignet sind über mehrere Trocknungs- oder Spülzyklen Riechstoffe in ausreichender Menge abzugeben, insbesondere nicht über fünf oder mehr Standard-Trocknungs- oder Spülzyklen.

Der wesentliche Nachteil derartiger Gegenstände mit limitierter Größe besteht neben der nur geringen Menge an Riechstoffen, die diese Gegenstände enthalten können, auch in der unpraktischen Anwendbarkeit durch den Konsumenten. Eine kleine Größe des Gegenstandes bedeutet für den Konsumenten, dass er nach erfolgter Benutzung des Gegenstandes in einem Wäschetrockner oder einer Geschirrspülmaschine diesen Gegenstand mühselig aus dem Gerät oder der Wäsche heraussuchen muss. Um den Komfort in der Benutzung der Gegenstände zu erhöhen, müssen Gegenstände bereitgestellt werden, die der zusätzlichen Anforderung einer für den Konsumenten angenehmen Größe entsprechen.

Die primäre Aufgabe der Erfindung war es demnach, einen mindestens 5 cm³ einnehmenden Gegenstand bereitzustellen, der für einen einmaligem, vorzugsweise für den mehrmaligen, Einsatz, vorzugsweise für einen mindestens fünffachen Einsatz, unter Standardzyklusbedingungen in einem Wäschetrockner und/oder Geschirrspüler geeignet ist, und einen ausreichend hohen Gewichtsanteil an einem oder mehreren Riechstoffen aufweist, wobei der Gegenstand vorzugsweise bei (wiederholter) Anwendung seine strukturelle Integrität nicht verliert, und wobei ein ausreichender Anteil der Riechstoffe während der Anwendung freigesetzt wird, so dass die Wäsche im Wäschetrockner bzw. die Luft im Geschirrspüler in ausreichendem Maße beduftet wird.

Gelöst wird die Aufgabe durch einen riechstoffhaltigen, einen einzelnen Hohlraum allseitig umschließenden Körper umfassend oder bestehend aus
- einer elastomeren, vorzugsweise vernetzten, Polymermatrix und
- einem oder mehreren in die Polymermatrix eingelagerten Riechstoffen in einer Gesamtmenge im Bereich von 10 bis 75 Gew.-%, bezogen auf das Gesamtgewicht des Hohlkörpers,
- gegebenenfalls weiteren in die Polymermatrix eingelagerten Stoffen,
   wobei
- der Hohlkörper ein Gesamtvolumen im Bereich von 5 bis 8181 CM³ bei 25°C und 1013 mbar besitzt,
   und
- die Dicke der den Hohlraum allseitig umschließenden Wandung an jeder Stelle im Bereich von 0,25 bis 20 mm liegt, bevorzugt im Bereich von 0,5 bis 15 mm, besonders bevorzugt im Bereich von 0,75 bis 10 mm, am meisten bevorzugt im Bereich von 1 bis 8 mm.

Ein erfindungsgemäßer Hohlkörper behält auch nach (mehrfacher) Verwendung im Wäschetrockner oder im Geschirrspüler seine strukturelle Integrität und ermöglicht eine ausreichende Beduftung in der jeweiligen Anwendung.

Ein erfindungsgemäßer Hohlkörper weist bevorzugt ein Gesamtvolumen im Bereich von 14 cm³ bis 905 cm³ auf, besonders bevorzugt im Bereich von 33 cm³ bis 524 cm³. Diese Volumina entsprechend in etwa einem Kugeldurchmesser im Bereich von 3 bis 12 cm bzw. im Bereich von 4 bis 10 cm. In diesen Bereichen ist eine Mehrfachanwendung problemlos möglich, der Platzbedarf des Hohlkörpers im Allgemeinen nicht zu hoch und eine ausreichende Stabilität sowie strukturelle Integrität gegeben.

Ein erfindungsgemäßer Hohlkörper weist vorzugsweise eine Gesamtmasse von zumindest 1 g auf, vorzugsweise eine Gesamtmasse im Bereich von 1 bis 100 g, bevorzugt im Bereich von 1 bis 50 g. Die bevorzugte Gesamtmasse hängt weitgehend vom Einsatzzweck und den Geräteabmessungen des Wäschetrockners bzw. Geschirrspülers sowie von der Anzahl der Wasch- oder Spülzyklen ab, die ein erfindungsgemäßer Hohlkörper absolvieren soll. So liegt die bevorzugte Gesamtmasse eines erfindungsgemäßen Hohlkörpers, beispielsweise für den Gebrauch in einem haushaltsüblichen Geschirrspüler, im Bereich von 1 bis 5 g. Die bevorzugte Gesamtmasse eines erfindungsgemäßen Hohlkörpers, beispielsweise für den Gebrauch in einem haushaltsüblichen Wäschetrockner, im Bereich von 1 bis 10 g, weiter bevorzugt im Bereich von 2 bis 8 g. In großen Geräten, wie beispielsweise kommerziell und industriell genutzten Wäschetrocknern hingegen, kann die optimale Gesamtmasse eines erfindungsgemäßen Hohlkörpers durchaus höher liegen, beispielsweise im Bereich von 5 bis 30 g.

Im Rahmen der vorliegenden Erfindung bedeutet es, wenn die Rede von in die Polymermatrix "eingelagerte" Stoffen, insbesondere Riechstoffen, ist, dass diese Stoffe in der Polymermatrix enthalten bzw. eingearbeitet sind und nicht in der Art vorliegen, dass sie einen Oberflächenfilm auf der Außenfläche und/oder der Innenwand des Hohlkörpers bilden

Im Rahmen der vorliegenden Erfindung wird als "Gesamtvolumen" das Außenvolumen des Hohlkörpers verstanden, also der insgesamt von dem Hohlkörper eingenommene Raum, einschließlich Wandung und des davon umschlossenen Hohlraumes.

Erfindungsgemäße Riechstoffe werden vorzugsweise ausgewählt aus der Gruppe bestehend aus (Angabe von branchenüblichen Produktnamen und Markennamen):

1-Phenyl-2-methyl-2-propylacetat, 2-Methylbutylbutyrat, Aldron (4-[(3,3-Dimethylbicyclo[2.2.1]hept-2-yl)methyl]-2-methylcyclohexanon), Allyl-2-cyclohexyloxyglycolat, Allyl-2-pentyloxyglycolat, Allyl-3-cyclohexylpropionat, Allylcapronat, Amarocit (1,1-Dimethoxy-2,2,5-trimethyl-4-hexen), Ambral (Dodecahydro-3,8,8,11a-tetramethyl-5H-3.5a-epoxynaphth[2.1-c]oxepin), Ambrettolid (9-Hexadecen-16-olid), Ambrinol S (1,2,3,4,4a,5,6,7-Octahydro-2,6,6-trimethyl-2-napthalinol), Ambrinolepoxid, Ambrocenide (4aR,5R,7aS,9R)-Octahydro-2,2,5,8,8,9a-hexamethyl-4H-4a,9-Methanoazuleno(5,6-d)-1,3-dioxol), Ambroxid (3a,6,6,9a-Tetramethyldodecahydronaphtho[2,1-b]-furan), Amylformiat, Aurelione (7-Cyclohexadecen-1-on und 8-Cyclohexadecen-1-on), Boronal [2-Methyl-4-(2,6,6-trimethyl-1-cyclohexen-1-yl)-butenal], Brahmanol [2-Methyl-4-(2,2,3-trimethyl-3-cyclopentenyl)-butanol], Buccoxime (1,5-Dimethylbicyclo[3.2.1]octan-8-onoxim), Butylacetat, Cantryl (2,2,3-Trimethyl-3-cyclopentenyl-1-acetonitril), Cassix 150 (4-Methoxy-2-methyl-2-butanthiol), Chrysantheme [1-(2,4-Dimethyl-3-cyclohexen-1-yl)-2,2-dimethyl-l-propanon], cis-3-Hexenylacetat, Citronellylbutyrat, Citronellyltiglinat (3,7-Dimethyl-6-octenyl-2-methylcrotonat), Citronitril (3-Methyl-5-phenyl-2-pentennitril), Citrowanil B (alpha-Ethenylalpha-methyl-benzolproprannitril), Claritone (2,4,7-Tetramethyl-6-octen-3-on), Corps Racine VS [2-(3-Phenylpropyl)pyridin], Coumarone (1-(2-Benzofuranyl)-ethanon), Cyclogalbanat (Allylcyclohexyloxyacetat), Cyclohexylmagnol (alpha-Methylcyclohexanpropanol), Datilat (1-Cyclohexylethylcrotonat), Ethyl-2-methylbutyrat, Ethylisobutyrat, Ethylisovalerat, Ethyltricyclo[5.2.1.0^{2,6}]decan-2-ylcarboxylat, Farenal (2,6,10-Trimethyl-9-undecenal), Filbertone (5-Methyl-2-hepten-4-on), Fleursandol (4-(3a,4,5,6,7,7a-Hexahydro-4,7-methano-1H-inden-6-yl)-3-methyl-3-buten-2-ol), Florazon (4-Ethyl-alpha,alpha-dimethyl benzolpropanal), Floropal (2,4,6-Trimethyl-4-phenyl-1,3-dioxan), Fragolane [(2,4-Dimethyl-[1,3]dioxolan-2-yl)essigsäureethylester)], Frutinat (But-2-ensäure-1,3-dimethylbutylester), gamma-Decalactone, Geranylacetat, Geranylbutyrat, Geranyltiglinat (trans-3,7-Dimethyl-2,6-octadienyl-2-methylcrotonat), Globalide [(11/12)-Pentadecen-15-olid], Globanone (8-Cyclohexadecen-1-on), Hexylbutyrat, Hydrocitronitril (beta-Methyl-benzolpentannitril), Indianol (4-[3a,4,5,6,7,7a-Hexahydro-4,7-methano-1H-inden-5(6)-yl]-3-methyl-3-buten-2-ol), Indoflor (4,4a,5,9b-Tetrahydoindeno[1,2-d]-m-dioxin), Irisnitril (2-Nonenylnitril), Isoamylacetat, Isoamylisovalerianat, Isodamascon [1-(2,4,4-Trimethyl-2-cyclohexen-1-yl)-2-buten-1-on], Isomuscon (Cyclohexadecanon), Jacinthaflor (2-Methyl-4-phenyl-1,3-dioxolan), Ketamber (Dodecahydro-3,8,8,11a-tetramethyl-5H-3,5a-epoxynaphth[2.1-c]oxepin), Lactojasmon (4-Hexyl-4-methyl-butyrolacton), Leguminal (Propanal-methyl-cis-3-hexenyl-acetal), Macrolide (Oxacyclohexadecan-2-on), Madranol (Mischung verschiedener Hexahydro methylionone), Magnolan (2,4-Dimethyl-5,6-indeno-1,3-dioxan), Majantol [2,2-Dimethyl-3-(3-methylphenyl)-propanol], Mandaril (3,12-Tridecadiennitril), Menthylacetat, Methylbutyrat, Methyldihydrojasmonat, Methylisobutyrat, Mintonat (3,3,5-Trimethylcyclohexylacetat), Mugetanol [1-(4-Isopropylcyclohexyl)-ethanol], Nerolione [1-(3-Methyl-2-benzofuranyl)-ethanon], Octylacetat, Ozonil (2-Tridecennitril), Palisandal (1,1-Dimethoxycyclododecan), Palisandin (Cyclododecylmethylether), Parmanyl [3-(cis-3-Hexenyloxy)-propannitril], Passifloran (3-Acetylthiohexylacetat), Peacholide (cis- und trans-3-Methyl-gamma-decalacton), Prenylsalicylat, Profarnesal (2,6,10-Trimethyl-5,9-undecadienal), Projasmon P (2-Heptylcyclopentanon), Pyroprunat (But-2-ensäurebicyclopenten-2-yl-ester), Rholiate (Kohlensäure-ethyl-2,3,6-trimethylcyclohexylester), Rosaphen (2-Methyl-5-phenylpentan-1-ol), Rosenoxid, Sandel 80 (trans-3-Isocamphylcyclohexanol), Sandranol (2-Ethyl-4-(2,2,3-trimethyl-3-cyclpenten-1-yl)-2-buten-l-ol), Symrose (4-Isoamylcyclohexanol), Symroxane (4-(3-Methylbutyl)-Cyclohexanol (Z)), Tabanon [4-(2-Butenyliden)-3,4,5-trimethyl-2-cyclohexen-1-on], Terpineol-4, Timberol (2,2,6-Trimethyl-alpha-propyl-cyclohexanpropanol), Tolylacetataldeyd D para (4-Methyl-Benzeneacetaldehyd), Tricyclodecenylpropionat, Tropicol (2-Mercapto-2-methyl-pentan-l-ol), Vertosine [2-(2,4-(oder 3,5)-Dimethyl-3-cyclohexen-1-yl)-methylenaminobenzoesäuremethylester], Vertral (Octahydro-4,7-methano-1H-indencarbaldehyd), Vetikolacetat (1,3-Dimethyl-3-phenylbutylacetat), Vetival (4-Cyclohexyl-4-methylpentan-2-on), Ysamber K (Spiro hexahydro-1',1',5',5'-tetramethyl-[1,3-dioxolan-2,8'-(5'H)-[2H-2,4a]-methanonaphthalin].

Weiter bevorzugte Riechstoffe, eingelagert in erfindungsgemäßen Hohlkörpern, sind genannt in S. Arctander, Perfume and Flavor Materials, Vol. I und II, Montclair, N. J. 1969, Eigenverlag, oder K. Bauer et al., Common Fragrance and Flavor Materials, 5th Edition, Wiley-VCH, Weinheim 2006.

Besonders bevorzugte erfindungsgemäße Hohlkörper enthalten vorzugsweise Extrakte aus natürlichen Rohstoffen wie ätherische Öle, Concretes, Absolues, Resine, Resinoide, Balsame, Tinkturen, wie z.B.

Ambratinktur; Amyrisöl; Angelicasamenöl; Angelicawurzelöl; Anisöl; Baldrianöl; Basilikumöl; Baummoos-Absolue; Bayöl; Beifussöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Teebaum-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen.

In einem weiter bevorzugten erfindungsgemäßen Hohlkörper enthält dieser einen, zwei oder mehrere Riechstoffe in Form von Einzelriechstoffen ausgewählt aus der Gruppe der Kohlenwasserstoffe. Besonders bevorzugt einzusetzende Einzelriechstoffe aus der Gruppe der Kohlenwasserstoffe sind z. B.

3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan; der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2;6-Dimethyl-5-heptenal; 10-Undecenal; (E)-4-Decenal; 2-Dodecena1;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen; der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on; der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol; der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril; der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat; 4-Methyl-2-pentyl-crotonat; der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate; der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal; der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate; der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetylierfes Cedernholzöl (Methylcedrylketon); der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol; der cycloaliphatischen Alkohole wie z.B. alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-l-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol; der cyclischen und cycloaliphatischen Ether wie z.B. Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan; der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon; der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd; der cycloaliphatischen Ketone wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton; der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat; der Ester cycloaliphatischer Alkohole wie z.B. 1-Cyclohexylethylcrotonat; der Ester cycloaliphatischer Carbonsäuren wie z.B. Allyl-3-cyclohexylpropionat; Allylcyclohexyloxyacetat; cis- und trans-Methyldihydrojasmonat; cis-und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat; der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol; der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin; der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal; der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon; (3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon; der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat; der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; 2-(3-Phenylpropyl)pyridin; Indol; Skatol; 2-Methoxy-3-isopropylpyrazin; 2-Isobutyl-3-methoxypyrazin; der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat; der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on; der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; cis- und trans-11-Pentadecen-1,15-olid; cis-und trans-12-Pentadecen-1,15-olid; 1,16-Hexadecanolid; 9-Hexadecen-1,16-olid; 10-Oxa-1,16-hexadecanolid; 11 -Oxa-1,16-hexadecanolid; 12-Oxa-1, 16-hexadecanolid; Ethylen-1,12-dodecandioat; Ethylen-1,13-tridecandioat; Cumarin; 2,3-Dihydrocumarin; Octahydrocumarin.

Die im Folgenden genannten Ausgestaltungen der Erfindung, insbesondere die (besonders) bevorzugten Ausgestaltungen und Ausführungsformen, erfüllen die gestellt Aufgabe in besonderem Maße. Die (besonders) bevorzugten erfindungsgemäßen Hohlkörper weisen bei Anwendung, auch bei wiederholter jeweiliger Anwendung, in einem Wäschetrockner oder Geschirrspüler eine besonders hohe Stabilität und strukturelle Integrität auf und beduften die Wäsche bzw. die Luft des Geschirrspülers in ausreichendem Maße.

Eine besonders gute strukturelle Integrität eines erfindungsgemäßen Hohlkörpers wird erzielt, wenn die als (besonders) bevorzugt bezeichneten Polyamine (insbesondere die unten beschriebenen Polyamid Polyamine), Vernetzer und/oder Stabilisatoren, insbesondere jeweils in den als bevorzugt angegebenen Mengenanteilen, eingesetzt werden. Ferner zeigen die erfindungsgemäßen Hohlkörper in den (besonders) bevorzugten Ausgestaltungen, ein über mehrere Zyklen ein im Wesentlichen gleichbleibendes Geruchsprofil, insbesondere im direkten Vergleich von zwei aufeinanderfolgenden Trocknungs- bzw. Spülzyklen. Dies gilt insbesondere, wenn die als (besonders) bevorzugt bezeichneten Riechstoffe, insbesondere in den als bevorzugt angegebenen Mengenanteilen, eingesetzt werden.

Vorzugsweise ist ein erfindungsgemäßer Hohlkörper für einen Einsatz, bevorzugt für einen mehrfachen Einsatz, in einem Wäschetrockner und/oder in einer Geschirrspülmaschine geeignet (Ausführungsform 1).

In einer bevorzugten Ausführungsform weist ein erfindungsgemäßer Hohlkörper nach 5 oder mehr, vorzugsweise nach 10 oder mehr, Standard-Trocknungszyklen in einem Wäschetrockner bzw. nach 10 oder mehr Standard-Spülzyklen in einem Geschirrspüler keine nennenswerten, vorzugsweise gar keine, strukturellen Schädigungen auf, d.h. der Hohlkörper übersteht die genannten Zyklen mit weitgehender, vorzugsweise vollständiger, struktureller Integrität (Ausführungsform 2).

Im Rahmen der vorliegenden Erfindung bedeutet "strukturelle Integrität", dass der zu untersuchende Gegenstand bzw. der erfindungsgemäße Hohlkörper nach ein- oder mehrfacher Anwendung in einem Wäschetrockner oder einem Geschirrspüler im Wesentlichen keine, vorzugsweise gar keine, mit dem bloßen Auge sichtbare Schädigung erlitten hat. Bevorzugt sind erfindungsgemäße Hohlkörper, die, bei einer zehnfachen optischen Vergrößerung, beispielsweise mit einem Lichtmikroskop, im Wesentlichen keine, vorzugsweise gar keine, sichtbare Schädigung erlitten haben.

Im Rahmen der vorliegenden Erfindung wird weiterhin ein Standard-Trocknungszyklus eines Wäschetrockners bzw. ein Standard-Spülzyklen eines Geschirrspülers wie folgt definiert:

### Standard-Trocknungszyklus Wäschetrockner:

4,5 kg Frotteehandtücher aus 90% Baumwolle und 10 % Viskose, welche zuvor bei 95°C mit einem kommerziell erhältlichen Flüssigwaschmittel gewaschen und bei 1100 U/min geschleudert worden waren wurden in den Wäschetrockner Miele Softronic T4222C eingebracht und bei dem Temperaturprogramm "Schranktrocken" getrocknet (Auslass-Temperatur des Trockners: 75°C).

### Standard-Spülzyklus Geschirrspüler:

Bosch Super Silence auto 3 in 1 (Temperaturprogramm "Auto 55-65°C"); Gesamtdauer eines Standard-Spülzyklus = ca. 2 Stunden und 19 Minuten.

In bevorzugten Ausgestaltungen betrifft die vorliegende Erfindung einen Hohlkörper, vorzugsweise gemäß einer der vorangehenden Ausführungsformen, wobei in die elastomere Polymermatrix als weitere Stoffe ein oder mehrere Stabilisatoren in einer Menge im Bereich von 5 bis 60 Gew.-%, vorzugsweise im Bereich von 10 bis 60 Gew.-%, weiter bevorzugt im Bereich von 20 bis 55 Gew.-%, besonders bevorzugt im Bereich von 30 bis 50 Gew.-%, eingelagert sind, jeweils bezogen auf das Gesamtgewicht des Hohlkörpers (Ausführungsform 3).

Besonders bevorzugt einzusetzende Stabilisatoren sind farbige Stabilisatoren oder Stoffe mit bestimmten Oberflächeneigenschaften. Diese besonders bevorzugten Stabilisatoren beeinflussen somit das Aussehen und die Textur des erfindungsgemäßen Hohlkörpers positiv, z.B. Farbe oder Textur der Oberfläche des Hohlkörpers.

Bevorzugte erfindungsgemäße Hohlkörper sind Hohlkörper nach Ausführungsform 3, wobei der, einer, mehrere oder sämtliche der Stabilisatoren bei 25°C und 1013 mbar gießfähig sind und einen Siedepunkt von größer oder gleich 180°C bei 1013 mbar, vorzugsweise einen Siedepunkt größer oder gleich 220°C bei 1013 mbar, bevorzugt einen Siedepunkt größer oder gleich 250°C bei 1013 mbar, aufweisen (Ausführungsform 4).

Im Rahmen der vorliegenden Erfindung bedeutet "gießfähig" sowohl flüssig, viskos als auch pastös, vorzugsweise flüssig. Jedoch ist mit "gießfähig" auch gemeint, dass in einer flüssigen, fließfähigen, gießbaren oder fließbaren Masse oder Flüssigkeit feste Anteile enthalten sein können, solange diese gegossen werden können.

Weiter bevorzugte erfindungsgemäße Hohlkörper sind Hohlkörper nach Ausführungsform 3 oder 4, wobei der, einer, mehrere oder sämtliche Stabilisatoren ausgewählt sind aus der Gruppe bestehend aus Phthalsäureestern, Paraffinen sowie Polyalkylenglykolen, Polyalkylenglykolethern und Polyalkylenglykolestern (Ausführungsform 5).

Besonders bevorzugte erfindungsgemäße Hohlkörper sind Hohlkörper nach einer der Ausführungsformen 3 bis 5, wobei der, einer, mehrere oder sämtliche der Stabilisatoren ausgewählt sind aus der Gruppe bestehend aus:
- Phthalsäureestern, vorzugsweise Diethylphthalat und Dibutylphthalat,
   und/oder
- Paraffinen, vorzugsweise lsoparaffinen, bevorzugt lsoparaffinen mit einem Siedepunkt im Bereich von 270 und 310°C bei 1013 mbar
   und/oder
- Polyalkylenglykolen, Polyalkylenglykolethern oder Polyalkylenglykolestern
   (Ausführungsform 6).

Die Polyalkylenglykole, deren Ether oder deren Ester weisen dabei vorzugsweise der folgende Formel auf:

R' ― O ― [CH₂ ― CHR ― O]ₙ ― R"

wobei gilt:
n = eine ganze Zahl im Bereich von 1 bis 50,
R = H oder Methyl,
R' = H, C₁-C₄-Alkyl, Acetyl, Phenyl,
R" = H, C₁-C₄-Alkyl, Acetyl, Phenyl
(Ausführungsform 6a).

Im Rahmen der Erfindung bevorzugt sind Polyalkylenglykole, deren Ether oder deren Ester entsprechend obiger Formel, wobei gilt:
n = 1, 2 oder 3,
und/oder
R = Methyl,
und/oder
R' = C₁-C₄-Alkyl,
und/oder
R" = H, C₁-C₄-Alkyl.

Im Rahmen der Erfindung als Stabilisatoren besonders bevorzugt sind dabei Polyalkylenglykolether, da diese eine besonders gute Stabilität und strukturelle lntegrität eines erfindungsgemäßen Hohlkörpers bewirken (Ausführungsform 6b).

Im Rahmen der Erfindung weiter bevorzugt sind Polyalkylenglykolether entsprechend obiger Formel, wobei gilt:
n = 2 oder 3,
R = Me,
R' = C₁-C₄-Alkyl,
und
R" = H, C₁-C₄-Alkyl (Ausführungsform 6c).

Ganz besonders bevorzugte erfindungsgemäße Hohlkörper sind Hohlkörper nach einer der Ausführungsformen 3 bis 6, wobei der, einer, mehrere oder sämtliche der Stabilisatoren ausgewählt sind aus der Gruppe bestehend aus Butyldiglykol, Dipropylenglycol-n-propylether, Propylenglykolphenylether, Dipropylenglykolmonomethylether, Tripropylenglykolmonomethylether, Dipropylenglykol-n-butylether und Tripropylenglykoln-butylether (Ausführungsform 7).

Stabilisatoren, die ausgewählt sind aus der Gruppe bestehend aus Phthalsäureestern, Paraffinen und Polyalkylenglykolen, deren Ethern oder deren Estern, sind für die Erfindung besonders bevorzugt, da sie, wenn sie sich in einem erfindungsgemäßen Hohlkörper befinden, in Trocknungszyklen von Wäschetrocknern und/oder Spülzyklen von Geschirrspülern nicht oder nur in geringem Maße verdampfen. Ein Verdampfen könnte zu Spannungen im Hohlkörper führen. Außerdem würden durch ein Verdampfen von Stabilisatoren Spannungen, die durch die Verringerung des Anteils der Riechstoffe durch deren Abgabe in die Wäsche oder Luft auftreten, noch verstärkt.

Erfindungsgemäß können alle Stabilisatoren eingesetzt werden, die sich im Polyamin der Matrix und in den Riechstoffen, die meist in Form eines Parfümöl vorliegen, lösen.

Ebenfalls bevorzugte erfindungsgemäße Hohlkörper sind Hohlkörper nach Ausführungsform 3, wobei der, einer, mehrere oder sämtliche der Stabilisatoren bei 25°C und 1013 3 mbar fest und ausgewählt sind aus der Gruppe bestehend aus Tonmineralen und anorganischen Pigmenten (Ausführungsform 8).

Ein erfindungsgemäßer Hohlkörper kann nicht transparente Stabilisatoren enthalten. Durch den Einsatz dieser nicht transparenten Stabilisatoren sind diese Ausgestaltungen des erfindungsgemäßen Hohlkörpers nicht mehr klar bzw. transparent formuliert.

Weiter bevorzugte erfindungsgemäße Hohlkörper sind Hohlkörper nach Ausführungsform 8, wobei der, einer, mehrere oder sämtliche Stabilisatoren ausgewählt sind aus der Gruppe bestehend aus Talkum, Kreide, Calciumsulfat, Calciumcarbonat, Bentonit, Zinkoxid, Titandioxid und Montmorillonit (Ausführungsform 9).

Erfindungsgemäß ganz besonders bevorzugt einzusetzende Stabilisatoren sind Stabilisatoren mit kleinen Korngrößen wie z. B. Talkum, Kreide, Zinkoxid oder Titandioxid. Weiter ganz besonders bevorzugte Stabilisatoren sind solche Stabilisatoren, die wirtschaftlich günstige Rohstoffe sind.

Bevorzugte erfindungsgemäße Hohlkörper, vorzugsweise Hohlkörper gemäß einer der vorangehenden Ausführungsformen, sind dadurch gekennzeichnet, dass als weiterer Stoff oder als weitere Stoffe in die elastomere Polymermatrix des Hohlkörpers ein oder mehrere Additivstoffe in einer Menge im Bereich von 0,5 bis 5 Gew.-%, vorzugsweise 1 bis 3 Gew.-%, jeweils bezogen auf das Gesamtgewicht des Hohlkörpers, eingelagert sind, und die ausgewählt sind aus der Gruppe bestehend aus:
- Farbstoffen,
- Glitter, und
- Stoffen, die zum Überdecken, Neutralisieren und/oder Verhindern schlechter Geruchseindrücke geeignet sind, jedoch keinen Eigengeruch aufweisen, dabei vorzugsweise Cyclodextrine (Ausführungsform 10).

Bevorzugte erfindungsgemäße Hohlkörper, vorzugsweise Hohlkörper gemäß einer der vorangehenden Ausführungsformen, sind dadurch gekennzeichnet, dass dieser 2, 3, 4, 5, 6, 7, 8, 9, 10 oder mehr Riechstoffe, vorzugsweise ein Parfümöl, enthält (Ausführungsform 11).

Bevorzugte erfindungsgemäße Hohlkörper, vorzugsweise Hohlkörper gemäß einer der vorangehenden Ausführungsformen, sind dadurch gekennzeichnet, dass die Gesamtmenge der in die elastomere Polymermatrix eingelagerten Riechstoffen im Bereich von 10 bis 50 Gew.-%, bevorzugt im Bereich von 10 bis 40 Gew.-% und besonders bevorzugt im Bereich von 15 bis 30 Gew.-%, liegt, jeweils bezogen auf das Gesamtgewicht des Hohlkörpers (Ausführungsform 12).

Bevorzugte erfindungsgemäße Hohlkörper, vorzugsweise Hohlkörper gemäß einer der vorangehenden Ausführungsformen, sind dadurch gekennzeichnet, dass zumindest einer, vorzugsweise zwei, weiter bevorzugt drei oder mehr der Riechstoffe einen log-K_{O}/_{W}, -Wert von kleiner als 4 aufweisen (Ausführungsform 13).

Der log-K_{O/W} -Wert ist ein Maß für die Polarität einer Substanz. Mit log-K_{O/W} wird der dekadische Logarithmus des Verteilungskoeffizienten einer Substanz zwischen 1-Octanol (unpolar) und Wasser (polar) bezeichnet.

Der log-K_{O/W} -Wert wird dabei in der Praxis rechnerisch ermittelt, wobei die jeweilige molekulare Struktur der Substanz, deren log-K_{O/W} -Wert bestimmt werden soll, zugrunde gelegt. Im Rahmen des vorliegenden Textes bezeichnet der Begriff "log-K_{o/w} -Wert" den anhand der jeweiligen molekularen strukturberechneten Wert bei Verwendung des Softwareprogrammes ,,EPlWlN" von P. Howard und W. Meylan [Version 2.2]. Dieses Softwareprogramm ist erhältlich bei Syracuse Research Corporation, Merrill Lane, Syracuse, NY 13210, USA.

Riechstoffe mit einem log-K_{O/W} -Wert von kleiner als 4 können in einem wässrigen Prozess nur sehr schwer auf ein Produkt übertragen werden. Diese Riechstoffe werden bei einem wässrigen Prozess, wie z. B. dem Wäschewaschen, beim Wäschetrocknen oder im Geschirrspüler, aufgrund ihrer Polarität mit dem Wasser fortgespült. Bevorzugt für die erfindungsgemäßen Hohlkörper einzusetzende Riechstoffe mit einem log-K_{O/W}-Wert sind gewählt aus der Gruppe (teils unter Angabe von branchenüblichen Produktnamen und Markennamen): FRUCTATE^{®}, MALTOL, ACETALDEHYD, ETHYLMALTOL, PHENOXYETHYLALKOHOL, ANISALKOHOL, JASMAPRUNAT, ETHYLPROPIONAT, FRAMBINON^{®}, COUMARIN, ETHYLVANILLIN, OCTAHYDROCOUMARIN, CIS-3-HEXENOL, VANILLIN, ANISYLNITRIL, FRAGOLANE^{®}, JACINTHAFLOR^{®}, ACETANISOL, HELIOTROPIN, INDOFLOR^{®}, ZIMTALKOHOL, CINNAMYLNITRIL, PHENYLETHYLALKOHOL, FLOROSA, BUCCOXIME^{®}, FILBERTONE, METHYLANTHRANILAT, HELIONAL, NEROLIONE, HYDROXYCITRONELLOL, DELTA-DECALACTON, GAMMA-DECALACTON, OCTENOL-1,3, ClS-3-HEXENYLACETAT, THUJON, ISOEUGENOL, MAGNOLAN, CYCLOGALBANAT^{®}, EUGENOL, MANZANATE, VETlKON^{®}, LlGUSTRAL, VERTOCITRAL, ISOBORNEOL, FENCHOL, BORNEOL, BETA-METHYLNAPHTYLKETON, MENTHON, ISOMENTHON, HEDIONE, PEACHOLlDE, ALDEHYD C16 SOGENANNT, PHENlRAT^{®}, MELONAL^{®}, IRISNITRIL, FLOROPAL, VERTACETAL^{®}, HERBAFLORAT, EVERNYL (Methyl-2-4-dihydroxy-3-6-dimethylbenzoat), NEROLIN YARA YARA, ClTROWANlL^{®} B, ALPHA-TERPINEOL, CANTRYL^{®}, FRESCOLAT^{®} ML (MENTHYLLACTAT), LlNALOOL, L-MENTHOL, ANETHOL, CITRAL, ROSAPHEN^{®}, GERANIOL, MAJANTOL^{®}, L-CITRONELLOL, CIS-ROSENOXID, MUGETANOL, VETIVAL^{®}, SYMROSE^{®}.

Bevorzugte erfindungsgemäße Hohlkörper, vorzugsweise Hohlkörper gemäß einer der vorangehenden Ausführungsformen, sind dadurch gekennzeichnet, dass der, einer, mehrere oder sämtliche der eingelagerten Riechstoffe ausgewählt sind aus der Gruppe bestehend aus FRUCTATE^{®}, MALTOL, ACETALDEHYD, ETHYLMALTOL, PHENOXYETHYLALKOHOL, ANISALKOHOL, JASMAPRUNAT, ETHYLPROPIONAT, FRAMBlNON^{®}, COUMARIN, ETHYLVANlLLlN, OCTAHYDROCOUMARIN, CIS-3-HEXENOL, VANILLIN, ANISYLNITRIL, FRAGOLANE^{®}, JAClNTHAFLOR^{®}, ACETANISOL, HELIOTROPIN, lNDOFLOR^{®}, ZIMTALKOHOL, CINNAMYLNITRIL, PHENYLETHYLALKOHOL, FLOROSA, BUCCOXlME^{®}, FILBERTONE, METHYLANTHRANILAT, HELlONAL, NEROLlONE, HYDROXYCITRONELLOL, DELTA-DECALACTON, GAMMA-DECALACTON, OCTENOL-1,3, CIS-3-HEXENYLACETAT, THUJON, ISOEUGENOL, MAGNOLAN, CYCLOGALBANAT^{®}, EUGENOL, MANZANATE, VETlKON^{®}, LlGUSTRAL, VERTOCITRAL, ISOBORNEOL, FENCHOL, BORNEOL, BETA-METHYLNAPHTYLKETON, MENTHON, ISOMENTHON, HEDIONE, PEACHOLlDE, ALDEHYD C16 SOGENANNT, PHENlRAT^{®}, MELONAL^{®}, IRISNITRIL, FLOROPAL, VERTACETAL^{®}, HERBAFLORAT, EVERNYL (Methyl-2-4-dihydroxy-3-6-dimethylbenzoat), NEROLIN YARA YARA, ClTROWANlL^{®} B, ALPHA-TERPINEOL, CANTRYL^{®}, FRESCOLAT^{®} ML (MENTHYLLACTAT), LlNALOOL, L-MENTHOL, ANETHOL, CITRAL, ROSAPHEN^{®}, GERANIOL, MAJANTOL^{®}, L-ClTRONELLOL, CIS-ROSENOXID, MUGETANOL, VETlVAL^{®}, SYMROSE^{®} (Ausführungsform 14).

Bevorzugte erfindungsgemäße Hohlkörper, vorzugsweise Hohlkörper gemäß einer der vorangehenden Ausführungsformen, sind dadurch gekennzeichnet, dass der Hohlkörper nach 5 Standard-Trocknungszyklen eines Wäschetrockners oder nach 5 Standard-Spülzyklen eines Geschirrspülers noch 40 Gew.-% oder mehr der Gesamtmenge der ursprünglich in dem Hohlkörper vorhandenen Riechstoffe enthält (Ausführungsform 15).

Bevorzugte erfindungsgemäße Hohlkörper, vorzugsweise Hohlkörper gemäß einer der vorangehenden Ausführungsformen, sind dadurch gekennzeichnet, dass höchstens 25 Gew.-% der Gesamtmenge der ursprünglich in dem Hohlkörper vorhandenen Riechstoffe einen Kovats-Index < 1500 aufweisen (Ausführungsform 16).

Der Anteil an Riechstoffen bzw. an Parfümöl im erfindungsgemäßen Hohlkörper wird durch den Fachmann je nach gewünschter Duftintensität gewählt. Vorzugsweise wird der Anteil der Riechstoffe bzw. des Parfümöls, enthaltend den oder die Riechstoffe, in dem erfindungsgemäßen Hohlkörper so gewählt, dass die Menge der Riechstoffe, die während eines Trocknungsvorgangs eines Wäschetrockners auf die Wäsche aufgebracht wird, geeignet ist, der Wäsche einen Duft zu vermitteln.

Bevorzugte erfindungsgemäße Hohlkörper, vorzugsweise Hohlkörper gemäß einer der vorangehenden Ausführungsformen, sind dadurch gekennzeichnet, dass pro Standard-Wäschetrockner- oder Standard-Geschirrspül-Zyklus je 50 mg oder mehr an Riechstoffen freigesetzt werden (Ausführungsform 17).

Eine solche Menge ist ausreichend, um die Wäsche in einem Wäschetrockner bzw. die Luft eines Geschirrspülers zu beduften.

Bevorzugte erfindungsgemäße Hohlkörper, vorzugsweise Hohlkörper gemäß einer der vorangehenden Ausführungsformen, sind dadurch gekennzeichnet, dass der Hohlkörper nahtlos ist und/oder die Form einer Kugel, eines Würfels, eines Zylinders, eines Kegels, eines Quaders, eines Ellipsoids oder eine kugelähnliche Form aufweist, bevorzugt sind Kugeln und kugelähnliche Formen (Ausführungsform 18).

Im Rahmen der vorliegenden Erfindung bedeutet "nahtlos" ohne Naht oder sichtbare Verbindungslinie. Es bedeutet zudem, dass der erfindungsgemäße Hohlkörper nicht dadurch hergestellt wird, dass zwei Teile separat hergestellt werden, die im Nachhinein über eine Naht, Verschmelzen oder ähnliches zusammengefügt werden.

Ganz besonders bevorzugt für einen erfindungsgemäßen Hohlkörper ist die Form einer (nahtlosen) Hohlkugel.

Die Erfindung schließt jedoch ebenso komplexe Formen wie Fische, Sterne oder Würfel ein. Bevorzugt sind jedoch Formen des erfindungsgemäßen Hohlkörpers, die weniger komplex sind. Durch weniger komplexe Formen, wie z. B. die Form einer Hohlkugel, ergibt sich eine homogenere Schichtdicke für den Hohlkörper. Eine homogenere Schichtdicke besitzt den Vorteil, dass die Gefahr des Brechens der Form minimiert wird.

Ganz besonders bevorzugt ist die Form einer Hohlkugel für den erfindungsgemäßen Hohlkörper, weil durch die sehr homogene Schichtdicke wenige Spannungen entstehen können. Ein erfindungsgemäßer Hohlkörper in Form einer Hohlkugel ist ein besonders stabiler Hohlkörper im Sinne der Erfindung.

Ein weiterer Vorteil, der in der Form einer Hohlkugel liegt, ist, dass durch die homogene Schichtdicke eine grundsätzlich gleichmäßigere Freisetzung der Riechstoffe möglich ist. Bei einem Vergleichshohlkörper in Form einer Vollkugel, d.h. einer Kugel, die nicht innen hohl ist, kommt es neben den Problemen des Zerberstens oder Zerbrechens auch zu dem weiteren Nachteil, dass die flüchtigen Wirkstoffe nicht gleichmäßig abgegeben werden, da sie zum Teil weitere Wege aus dem inneren der Kugel an den Rand zurücklegen müssen.

Bevorzugte erfindungsgemäße Hohlkörper, vorzugsweise Hohlkörper gemäß einer der vorangehenden Ausführungsformen, sind dadurch gekennzeichnet, dass die elastomere Polymermatrix kalt vernetzt ist (Ausführungsform 19).

Bevorzugte erfindungsgemäße Hohlkörper, vorzugsweise Hohlkörper gemäß einer der vorangehenden Ausführungsformen, sind dadurch gekennzeichnet, dass die elastomere Polymermatrix das Reaktionsprodukt
i) eines Vernetzers aus der Gruppe der Epoxide, Isocyanate, Anhydride oder Acrylate
   mit
ii) einem, zwei oder mehr Polyaminen
   ist oder enthält (Ausführungsform 20).

Bevorzugte erfindungsgemäße Hohlkörper, vorzugsweise Hohlkörper gemäß einer der vorangehenden Ausführungsformen, sind dadurch gekennzeichnet, dass die elastomere Polymermatrix das Reaktionsprodukt von
i) 2 bis 5 Gew.-% eines Vernetzers aus der Gruppe der Epoxide, Isocyanate, Anhydride oder Acrylate, vorzugsweise 2 bis 5 Gew.-% eines Vernetzers aus der Gruppe der Isocyanate,
   mit
ii) insgesamt 10 bis 35 Gew.-%, vorzugsweise 15 bis 30 Gew.-% eines, zweier oder mehr Polyamine
ist oder enthält,
wobei die Gew.-%-Angaben jeweils bezogen sind auf das Gesamtgewicht des Hohlkörpers (Ausführungsform 21).

Bei bestimmten Ausführungsformen des erfindungsgemäßen Hohlkörpers ist die Polymermatrix mit einem Stoff mit mindestens einer Expoxidgruppe als Vernetzerkomponente gebildet. Erfindungsgemäß bevorzugt besitzt die Vernetzerkomponente für die Herstellung eines erfindungsgemäßen Hohlkörpers, wenn die Vernetzerkomponente eine Epoxykomponente ist, mehrere Expoxygruppen.

Besonders bevorzugt für die Herstellung einer Polymermatrix für einen erfindungsgemäßen Hohlkörper wird eine Epoxykomponente benutzt, die bei Raumtemperatur und 1013 3 mbar in flüssiger Form vorliegt. Bevorzugt einzusetzende Epoxide sind im "Handbook of epoxy resins" von Henry Lee und Kris Neville (McGraw Hill, 1967), "The Epoxy Formulators Manual" der Society of Plastics Industry, Inc. (1984), und der "Encyclopedia of Science and Technology" (Kirk-Othmer, John Wiley & Sons, 1994) aufgeführt.

Ganz besonders bevorzugte flüssige Epoxydharze als Ausgangsstoff für die Herstellung einer Polymermatrix für einen erfindungsgemäßen Festkörper sind Diglycidylester von Bisphenol A und F (erhältlich als EPON 828 und EOIB 8620 von Resolution Performance Products), hydrierte Glycidylether von Bisphenol A (erhältlich als EPALLOY 5000 und EPALLOY 5001 von CVE Speciality Chemicals), die Diglycidylether von Butandiol, Dimethanolcyclohexan, Neopenthylglykol und Trimethylpolypropan (alle erhältlich in der HELOSY Modifier Produktlinie bei Resolution Performance Products).

Bei bestimmten Ausführungsformen des erfindungsgemäßen Hohlkörpers wird die Polymermatrix mit einer Vernetzerkomponente hergestellt, die mindestens eine funktionelle Isocyanatgruppe enthält. Erfindungsgemäß bevorzugt enthält eine derartige lsocyanatvenetzerkomponente mehrere Isocyanatgruppen und entspricht einem Polyisocyanat. Weiter bevorzugt ist die lsocyanatvenetzerkomponente bei 25°C und 1013 mbar flüssig.

Besonders bevorzugte Isocyanate im Rahmen dieser Erfindung sind aliphatische difunktionale Isocyanate (zum Beispiel lsophorondiisocyanat oder Bis(4-isocyanatocyclohexyl) methan.

Ganz besonders bevorzugt für die Herstellung einer Polymermatrix eines erfindungsgemäßen Hohlkörpers sind polyfunktionale Isocyanate mit einer Siedetemperatur von über 180°C bei 1013 mbar und reduzierter Toxizität. Beispiele für diese ganz besonders bevorzugten polyfunktionalen Isocyanate sind die von Bayer erhältlichen Produkte der DESMODUR Reihe. In der DESMODUR Reihe ist besonders die DESMODUR N Serie aliphatischer Isocyanate bevorzugt, dabei besonders DESMODUR N-3300 und N-3800, sowie die DESMODUR Z Serie, dort insbesondere DESMODUR Z4470.

Bei bestimmten Ausführungsformen des erfindungsgemäßen Hohlkörpers wird die Polymermatrix mit einer Vernetzerkomponente hergestellt, die mindestens eine funktionelle Anhydridgruppe besitzt. Bevorzugt einzusetzende Anhydride besitzen mehrere funktionelle Anhydridgruppen (Polyanhydride). Weiter sind bevorzugt Anhydride einzusetzen, die bei 25°C und 1013 mbar flüssig vorliegen.

Ganz besonders bevorzugt einzusetzen als Vernetzerkomponente für die Herstellung einer Polymermatrix für einen erfindungsgemäßen Festkörper sind Anhydride, die Kunststoffe sind, die nicht auf Maleinsäure basieren.

Bei bestimmten Ausführungsformen des erfindungsgemäßen Hohlkörpers enthält dieser eine Polymermatrix, bei deren Herstellung die Vernetzerkomponente ein Stoff mit mindestens einer funktionellen Acrylatgruppe ist. Bevorzugt einzusetzende Acrylate als Vernetzerkomponente besitzen mehrere Acrylatgruppen (Polyacrylate). Ganz besonders bevorzugt werden Polyacrylate im Sinne der Erfindung eingesetzt, wenn sie bei 25°C und 1013 mbar flüssig sind.

Alle aufgeführten Expoxide, Isocyanate, Anhydride und Acrylate sind repräsentativ und beschränken die Ausführung der Erfindung nicht auf dieselbigen.

Weiter bevorzugte erfindungsgemäße Hohlkörper sind Hohlkörper nach Ausführungsform 20 oder 21, dadurch gekennzeichnet, dass das, ein, beide oder mehrere der Polyamine ein Polyamid-Polyamin, vorzugsweise ein Polyamid-Polyether-Blockpolymer-Polyamin sind (Ausführungsform 22).

Besonders bevorzugte erfindungsgemäße Hohlkörper sind Hohlkörper nach einer der Ausführungsformen 20 bis 22, dadurch gekennzeichnet, dass das, ein, beide oder mehrere der Polyamine bei 25°C und 1013 mbar gießfähig, vorzugsweise flüssig, sind (Ausführungsform 23).

Weiter besonders bevorzugte erfindungsgemäße Hohlkörper sind Hohlkörper nach einer der Ausführungsformen 20 bis 23, dadurch gekennzeichnet, dass das, ein, beide oder mehrere der Polyamine aliphatisch sind (Ausführungsform 24).

Für die Erfindung einzusetzende ganz besonders bevorzugte Polyamine oder Mischungen aus Polyaminen sind nicht aromatisch.

Ganz besonders bevorzugte erfindungsgemäße Hohlkörper sind Hohlkörper nach einer der Ausführungsformen 20 bis 24, dadurch gekennzeichnet, dass die mittlere Molmasse M_{W} (gewichtsmittlere Molmasse) der Gesamtmenge der ii) Polyamine im Bereich von 5000 bis 15000 g/mol liegt (Ausführungsform 25).

Weiter ganz besonders bevorzugte erfindungsgemäße Hohlkörper sind Hohlkörper nach einer der Ausführungsformen 20 bis 25, dadurch gekennzeichnet, dass ii) zwei oder mehr verschiedene Polyamine aufweist, wobei zumindest ein Polyamin eine mittlere Molmasse M_{W} (gewichtsmittlere Molmasse) im Bereich von 5000 bis 8000 g/mol und zumindest ein weiteres Polyamin eine mittlere Molmasse M_{W} (gewichtsmittlere Molmasse) on 9000 bis 12000 g/mol besitzt (Ausführungsform 26).

Beispiele für erfindungsgemäß einzusetzende Polyamine sind JEFFAMINE D-230, T-403 oder XTJ-511 der Huntsman Corporation. Weitere erfindungsgemäß einzusetzende Polyamine sind cycloaliphatische Diamine wie Isophorondiamin (lPDA), Aminoethylpiperazin und 1.3-Bis(aminomethyl)cyclohexan (1,3-BAC) und Polyamine der Polyamidoaminofamilie z. B. die UNIREZ Serie von Arizona Chemicals.

Ferner können die Polyamine bei der vorliegenden Erfindung eingesetzt werden, die in den Patentanmeldungen WO 2005/118008, US 2005/0267231 und/oder WO 2008/079892 und/oder in den US Patenten US 6,870,011 und/oder US 6,399,713 beschrieben sind, welche hiermit ausdrücklich im Wege der Verweisung Bestandteil der vorliegenden Anmeldung und der (bevorzugten bzw. besonders bevorzugten) Ausgestaltungen werden. Die dort bevorzugt genannten Polyamine, vorzugsweise die dort genannten Polyamid Polyamine, sowie die dort genannten Vernetzer sind ausdrücklich und jeweils explizit im Rahmen der vorliegenden Erfindung ebenfalls bevorzugt.

Insbesondere die in WO 2005/118008, US 2005/0267231 sowie WO 2008/079892 explizit beschriebenen sowie bevorzugten Polyamine und Vernetzer sind im Rahmen der vorliegenden Erfindung ebenfalls bevorzugt, da mit diesen Polyaminen und/oder Vernetzern die der vorliegenden Erfindung zu Grunde liegende Aufgabe in besonderem Maße gelöst werden kann.

Für die Herstellung einer Polymermatrix eines erfindungsgemäßen Hohlkörpers kann das Polyamin auch als Mischung aus zwei oder mehreren Polyaminen vorliegen. Mit der Wahl der Mischung bestimmter Polyamine kann der Fachmann Einfluss auf die Viskosität, die Härtezeit und Produkteigenschaften nehmen. Vorteilhafte Polyaminmischungen sind für den Fachmann leicht erkennbar und des Weiteren werden beispielhafte Polyaminmischungen in den nachfolgenden Beispielen offenbart.

Erfindungsgemäße riechstoffhaltige Hohlkörper können dadurch gekennzeichnet sein, dass das oder die Polyamid-Polyamine hergestellt sind aus nicht-aromatischen Di- oder Triaminen, bevorzugt aus primären Di- oder Triaminen oder sekundären Diaminen, weiter bevorzugt aus primären Polyalkylenoxydi- oder triaminen, dabei vorzugsweise mit einer Molmasse von 200 bis 5000 Dalton, oder Piperazin.

Weiterhin können erfindungsgemäße riechstoffhaltige Hohlkörper dadurch gekennzeichnet sein, dass das oder die Polyamine ein Polyamid-Polyether-Blockpolymer-Polyamin ist, das das Reaktionsprodukt aus einem oder mehreren Polyalkylenoxy-Polyaminen mit einer oder mehreren aliphatischen Polysäuren ist.

Bevorzugt einzusetzende Polysäuren sind dabei ausgewählt sind aus der Gruppe bestehend aus dimerisierter Ölsäure, 1,4-Cyclohexandicarbonsäure, hydrierter Dimersäure und Adipinsäure.

Bei einem erfindungsgemäßen riechstoffhaltigen Hohlkörper liegt der Anteil des Volumens des Hohlraums, bezogen auf das Gesamtvolumen des Hohlkörpers, in einem Bereich zwischen einem Vielfachen des Volumens der Polymermatrix und einem Anteil an dem Volumen der Polymermatrix.

Bevorzugte erfindungsgemäße Hohlkörper, vorzugsweise Hohlkörper gemäß einer der vorangehenden Ausführungsformen, sind dadurch gekennzeichnet, dass das Volumen des Hohlraums im Bereich von 40 Vol.-% bis 98 Vol.-% , bevorzugt im Bereich von 70 Vol.-% bis 95 Vol.-% liegt, bezogen auf das gesamte Volumen des riechstoffhaltigen Hohlkörpers bei 25°C und 1013 mbar (Ausführungsform 27).

Eine erfindungsgemäße Verwendung eines erfindungsgemäßen Hohlkörpers, vorzugsweise in Ausgestaltung einer der vorangehenden Ausführungsformen 1 bis 27, besteht in der Aufbringung eines Duftes auf Wäsche während eines Trockenvorganges in einem Wäschetrockner und/oder in der Beduftung von Geschirrspülerluft während eines Spülvorganges eines Geschirrspülers, wobei der erfindungsgemäße Hohlkörper für einen mehrmaligen Gebrauch geeignet ist.

Eine bevorzugte erfindungsgemäße Verwendung ist dadurch gekennzeichnet, dass der Hohlkörper nach mehrmaligem Gebrauch (vorzugsweise nach 5 oder mehr, bevorzugt nach 10 oder mehr Standard-Zyklen) weitgehende oder vollständige strukturelle Integrität aufweist.

Ein erfindungsgemäßes Verfahren zur Herstellung eines erfindungsgemäßen Hohlkörpers, vorzugsweise gemäß einer der vorangehenden Ausführungsformen 1 bis 27, umfasst oder besteht aus folgenden Schritten:
a) Bereitstellen eines oder mehrerer Edukte zur Herstellung einer elastomeren, vorzugsweise vernetzten Polymermatrix,
b) Zugeben von 10 bis 75 Gew.-% eines oder mehrerer Riechstoffe,
c) Gegebenenfalls Zugeben von 5 bis 60 Gew.-%, vorzugsweise in einem Bereich von 10 bis 60 Gew.-%, bevorzugt in einem Bereich von 20 bis 55 Gew.-%, weiter bevorzugt in einem Bereich von 30 bis 50 Gew.-%, Stabilisatoren sowie gegebenenfalls weiterer Bestandteile,
d) Mischen von Edukten, Riechstoffen, gegebenenfalls Stabilisatoren und gegebenenfalls weiteren Bestandteilen, so dass die elastomere, vorzugsweise vernetzte Polymermatrix gebildet wird,
e) Ausformen eines Hohlkörpers, vorzugsweise durch ein Schleuderverfahren unter Einsatz der in Schritt d) erzeugten Mischung,
wobei die Gew.-%-Angaben jeweils bezogen sind auf das Gesamtgewicht des Hohlkörpers.

Ein bevorzugtes erfindungsgemäßes Verfahren ist dadurch gekennzeichnet, dass der Vernetzer ein Stoff mit mindestens einer funktionellen Gruppe ist ausgewählt aus der Gruppe bestehend aus Epoxiden, Isocyanaten, Anhydriden und Acrylaten.

Ein besonders bevorzugtes Verfahren zur Herstellung eines erfindungsgemäßen Hohlkörpers benutzt als Vernetzer DESMODUR N-3300.

Ein weiterhin besonders bevorzugtes erfindungsgemäßes Verfahren zur Herstellung eines erfindungsgemäßen Hohlkörpers benutzt zur Ausformung einer Hohlkugel ein Schleuderverfahren, bei dem die Drehgeschwindigkeit so reguliert ist, dass ein "Verlaufen" der Polymer-Matrix noch möglich ist. Es bleibt dem Fachmann überlassen eine entsprechende Drehgeschwindigkeit zu wählen, so dass die Beschichtung nicht einseitig erfolgt (eine einseitige Beschichtung wäre bei zu niedrigen Drehzahlen zu befürchten) und so dass eine gleichmäßige Beschichtung gewährleistet ist (bei zu hohen Drehzahlen ist eine gleichmäßige Beschichtung nicht gewährleistet). Der Fachmann orientiert sich dabei an Verfahren, die ihm z.B. aus der Herstellung von Weihnachtsbaumkugeln bekannt sind.

Für die erfindungsgemäßen Verwendungen und die erfindungsgemäßen Verfahren gelten die jeweiligen oben beschriebenen (bevorzugten bzw. besonders bevorzugten) Ausführungsformen der erfindungsgemäßen Hohlkörper entsprechend.

Bei quantitativen Betrachtungen der Zusammensetzung erfindungsgemäßer Hohlkörper wird ein enthaltener Stoff, wenn er zwei Bestandteilen zugerechnet werden kann, dem Bestandteil zugerechnet, der oben zuerst genannt wurde (z.B.: handelt es sich bei dem Stoff um einen Riechstoff, der zudem ein Stabilisator ist, wird dieser Stoff den Riechstoffen zugeordnet).

Die nachfolgenden Beispiele geben einige erfindungsgemäße Hohlkörper an.

Die Bezeichnungen Sylvaclear^{®} und Dowanol^{®} sind Markennamen der jeweiligen Hersteller.

### Beispiel 1

| Inhaltsstoffe | Chemische Bezeichnung / Klassifizierung | Gewichtsprozent [%] |
|---|---|---|
| | | |
| 188178 Blues | Parfümöl (Symrise) | 20,00 |
| Dowanol DPM | 1-(2-Methoxypropoxy)-2-Propanol | ad 100,00 |
| Sylvaclear IM 700 | Polyamin | 26,40 |
| Sylvaclear IM 800 | Polyamin | 0,031 |
| D&C Red No. 10 | 1-(4-(Penylazo)phenylazo)-2-naphtol | 1,50 |
| Desmodur N-3300 | Aliphatisches Isocyanat | 3,60 |

### Beispiel 2

| Inhaltsstoffe | Chemische Bezeichnung / Klassifizierung | Gewichtsprozent [%] |
|---|---|---|
| | | |
| 382065 Sensitiv | Parfümöl (Symrise) | 20,00 |
| Dowanol DPM | 1-(2-Methoxypropoxy)-2-Propanol | ad 100,00 |
| Sylvaclear IM 700 | Polyamin | 10,40 |
| Sylvaclear IM 800 | Polyamin | 16,40 |
| Titandioxid | Titandioxid | 1,50 |
| Talkum | Talkum | 9,00 |
| Desmodur N-3300 | Aliphatisches Isocyanat | 3,60 |

### Beispiel 3

| Inhaltsstoffe | Chemische Bezeichnung / Klassifizierung | Gewichtsprozent [%] |
|---|---|---|
| | | |
| 228151 Lemonfreak | Parfümöl (Symrise) | 30,00 |
| Dowanol TPnB | Tripropylenglycol-n-butylether | ad 100,00 |
| Sylvaclear IM 700 | Polyamin | 13,20 |
| Sylvaclear IM 800 | Polyamin | 13,20 |
| Perlglanzpigment Gold BB | Pigment (Merck) | 0,10 |
| Desmodur N-3300 | Aliphatisches Isocyanat | 3,60 |

### Herstellung:

Die Herstellung der hohlen Körpers erfolgt durch Mischen der aller Komponenten bis auf den Vernetzer (z.B. Desmodur N-3300). Nach der Zugabe des Vernetzers muss noch einmal schnell und gründlich gemischt werden. Je nach Parfümöl und der Mischung der Polymerkomponenten erfolgt die Härtung mehr oder weniger schnell.

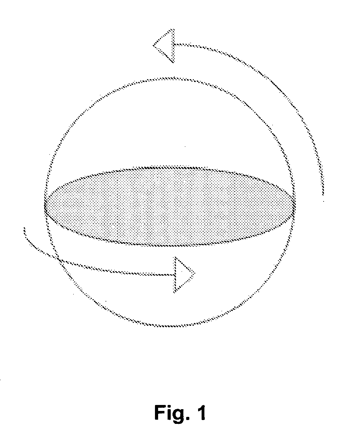

Zur Herstellung dieses holen Körpers ist es erforderlich, den Körper innen an allen Stellen möglichst gleich dick zu beschichten. Dabei entsteht in der Mitte des Körpers ein luftgefüllter Raum. Die Stabilität des Hohlkörpers kann über die Schichtdicke mit beeinflusst werden. Die Schichtdicke wird über das Verhältnis eingefüllte Polymermatrixmenge zu Formdurchmesser geregelt. Als Form für die Hohlform dient z.B. eine Polyacrylatform oder Polyethylenform die in der Mitte an einer Schnittkante getrennt wird. Befüllt wird eine Halbform mit der Polymermatrix. Anschließend werden die beiden Halbformen zu der kompletten Form zusammengefügt und in eine Maschine eingespannt. Die Maschine versetzt die Kugelform mit der eingefüllten Polymermatrix gleichzeitig über mindestens 2 Achsen in Rotation (vergleiche Fig. 1).

Die Drehgeschwindigkeit ist hierbei so zu regulieren, dass ein "verlaufen" der Polymermatrix noch möglich ist. Bei zu hohen Geschwindigkeiten wird eine gleichmäßige Beschichtung nicht gewährleistet. Bei zu niedrigen Drehzahlen ist die Beschichtung einseitig. Die Rotation wird während des kompletten Aushärteprozesses beibehalten. Nach abgeschlossenem Aushärteprozess wird die Form an der Schnittkante getrennt und die Polymerform wird entnommen.

## Patentansprüche

1. Riechstoffhaltiger, einen einzelnen Hohlraum allseitig umschließender Körper umfassend oder bestehend aus
- einer elastomeren, vorzugsweise vernetzten, Polymermatrix und
- einem oder mehreren in die Polymermatrix eingelagerten Riechstoffen in einer Gesamtmenge im Bereich von 10 bis 75 Gew.-%, bezogen auf das Gesamtgewicht des Hohlkörpers,
- gegebenenfalls weiteren in die Polymermatrix eingelagerten Stoffen,
wobei
- der Hohlkörper ein Gesamtvolumen im Bereich von 5 bis 8181 cm³ bei 25°C und 1013 mbar besitzt,
und
- die Dicke der den Hohlraum allseitig umschließenden Wandung an jeder Stelle im Bereich von 0,25 bis 20 mm liegt, bevorzugt im Bereich von 0,5 bis 15 mm, besonders bevorzugt im Bereich von 0,75 bis 10 mm, am meisten bevorzugt im Bereich von 1 bis 8 mm.

2. Riechstoffhaltiger Hohlkörper nach Anspruch 1, wobei der Hohlkörper für den Einsatz, vorzugsweise für einen mehrfachen Einsatz, in einem Wäschetrockner und/oder in einer Geschirrspülmaschine geeignet ist.

3. Riechstoffhaltiger Hohlkörper nach einem der vorangehenden Ansprüche, **dadurch gekennzeichnet, dass** der Hohlkörper 5 oder mehr, vorzugsweise 10 oder mehr Standard-Trocknungszyklen eines Wäschetrockners oder 10 oder mehr Standard-Spülzyklen eines Geschirrspülers mit struktureller Integrität übersteht.

4. Riechstoffhaltiger Hohlkörper nach einem der vorangehenden Ansprüche, wobei in die elastomere Polymermatrix als weitere Stoffe ein oder mehrere Stabilisatoren in einer Menge im Bereich von 5 bis 60 Gew.-%, vorzugsweise im Bereich von 10 bis 60 Gew.-%, weiter bevorzugt im Bereich von 20 bis 55 Gew.-%, besonders bevorzugt im Bereich von 30 bis 50 Gew.-%, eingelagert sind, jeweils bezogen auf das Gesamtgewicht des Hohlkörpers.

5. Riechstoffhaltiger Hohlkörper nach Anspruch 4, wobei der, einer, mehrere oder sämtliche der Stabilisatoren bei 25°C und 1013 mbar
gießfähig sind und einen Siedepunkt von größer oder gleich 180°C bei 1013 mbar, vorzugsweise einen Siedepunkt größer oder gleich 220°C bei 1013 mbar, bevorzugt einen Siedepunkt größer oder gleich 250°C bei 1013 mbar aufweisen und vorzugsweise ausgewählt sind aus der Gruppe bestehend aus Phthalsäureestern, Paraffinen, Polyalkylenglykolen, Polyalkylenglykolethern oder Polyalkylenglykolestern,
und/oder
fest und ausgewählt sind aus der Gruppe bestehend aus Tonmineralen und anorganischen Pigmenten und vorzugsweise ausgewählt sind aus der Gruppe bestehend aus Talkum, Kreide, Calciumsulfat, Calciumcarbonat, Bentonit, Zinkoxid, Titandioxid und Montmorillonit.

6. Riechstoffhaltiger Hohlkörper nach einem der vorangehenden Ansprüche, **dadurch gekennzeichnet, dass** die Gesamtmenge der in die elastomere Polymermatrix eingelagerten Riechstoffen im Bereich von 10 bis 50 Gew.-%, bevorzugt im Bereich von 10 bis 40 Gew.-% und besonders bevorzugt im Bereich von 15 bis 30 Gew.-%, liegt, jeweils bezogen auf das Gesamtgewicht des Hohlkörpers.

7. Riechstoffhaltiger Hohlkörper nach einem der vorangehenden Ansprüche, **dadurch gekennzeichnet, dass** der Hohlkörper nach 5 Standard-Trocknungszyklen eines Wäschetrockners oder nach 5 Standard-Spülzyklen eines Geschirrspülers noch 40 Gew.-% oder mehr der Gesamtmenge der ursprünglich in dem Hohlkörper vorhandenen Riechstoffe enthält.

8. Riechstoffhaltiger Hohlkörper nach einem der vorangehenden Ansprüche, **dadurch gekennzeichnet, dass** pro Standard-Wäschetrockner- oder Standard-Geschirrspül-Zyklus je 50 mg oder mehr an Riechstoffen freigesetzt werden.

9. Riechstoffhaltiger Hohlkörper nach einem der vorangehenden Ansprüche, **dadurch gekennzeichnet, dass** die elastomere Polymermatrix kalt vernetzt ist.

10. Riechstoffhaltiger Hohlkörper nach einem der vorangehenden Ansprüche, **dadurch gekennzeichnet, dass** die elastomere Polymermatrix das Reaktionsprodukt
i) eines Vernetzers aus der Gruppe der Epoxide, Isocyanate, Anhydride oder Acrylate
mit
ii) einem, zwei oder mehr Polyaminen
ist oder enthält.

11. Riechstoffhaltiger Hohlkörper nach einem der vorangehenden Ansprüche, **dadurch gekennzeichnet, dass** die elastomere Polymermatrix das Reaktionsprodukt von
i) 2-5 Gew.-% eines Vernetzers aus der Gruppe der Epoxide, Isocyanate, Anhydride oder Acrylate, vorzugsweise 2-5 Gew.-% eines Vernetzers aus der Gruppe der Isocyanate,
mit
ii) insgesamt 10 bis 35 Gew.-%, vorzugsweise 15 bis 30 Gew.-% eines, zweier oder mehr Polyamine
ist oder enthält,
wobei die Gew.-%-Angaben jeweils bezogen sind auf das Gesamtgewicht des Hohlkörpers.

12. Riechstoffhaltiger Hohlkörper nach Anspruch 10 oder 11, **dadurch gekennzeichnet, dass** das, ein, beide oder mehrere der Polyamine bei 25°C und 1013 mbar gießfähig, vorzugsweise flüssig, sind.

13. Riechstoffhaltiger Hohlkörper nach einem der vorangehenden Ansprüche, **dadurch gekennzeichnet, dass** das Volumen des Hohlraums im Bereich von 40 Vol.-% bis 98 Vol.-%, bevorzugt im Bereich von 70 Vol.-% bis 95 Vol.-% liegt, bezogen auf das gesamte Volumen des riechstoffhaltigen Hohlkörpers bei 25°C und 1013 mbar.

14. Verwendung eines riechstoffhaltigen Hohlkörpers gemäß einem der Ansprüche 1 bis 13 für die Aufbringung eines Duftes auf die Wäsche während eines Trockenvorganges in einem Wäschetrockner und/oder für die Beduftung der Geschirrspülerluft während eines Spülvorganges eines Geschirrspülers, wobei der Hohlkörper für einen mehrmaligen Gebrauch geeignet ist.

15. Verfahren zur Herstellung eines riechstoffhaltigen Hohlkörpers nach einem der Ansprüche 1 bis 13
umfassend oder bestehend aus folgenden Schritten:
a) Bereitstellen eines oder mehrerer Edukte zur Herstellung einer elastomeren, vorzugsweise vernetzten Polymermatrix,
b) Zugeben von 10 bis 75 Gew.-% eines oder mehrerer Riechstoffe,
c) Gegebenenfalls Zugeben von 5 bis 60 Gew.-%, vorzugsweise in einem Bereich von 10 bis 60 Gew.-%, bevorzugt in einem Bereich von 20 bis 55 Gew.-%, weiter bevorzugt in einem Bereich von 30 bis 50 Gew.-%, Stabilisatoren sowie gegebenenfalls weiterer Bestandteile,
d) Mischen von Edukten, Riechstoffen, gegebenenfalls Stabilisatoren und gegebenenfalls weiteren Bestandteilen, so dass die elastomere, vorzugsweise vernetzte Polymermatrix gebildet wird,
e) Ausformen eines Hohlkörpers, vorzugsweise durch ein Schleuderverfahren unter Einsatz der in Schritt d) erzeugten Mischung,
wobei die Gew.-%-Angaben jeweils bezogen sind auf das Gesamtgewicht des Hohlkörpers.

## Claims

1. Fragrance-containing body, enclosing a single cavity on all sides, comprising or consisting of
- an elastomeric, preferably cross-linked, polymer matrix and
- one or more fragrances embedded in the polymer matrix in a total amount in the range from 10 to 75 wt.-% with respect to the total weight of the hollow body,
- optionally further substances embedded in the polymer matrix,
wherein
- the hollow body has a total volume in the range from 5 to 8181 cm³ at 25 °C and 1013 mbar,
and
- the thickness of the wall enclosing the cavity on all sides is in the range from 0.25 to 20 mm, preferably in the range from 0.5 to 15 mm, particularly preferably in the range from 0.75 to 10 mm, most preferably in the range from 1 to 8 mm, at any position.

2. Fragrance-containing hollow body according to claim 1, wherein the hollow body is suitable for use, preferably for multiple use, in a tumble dryer and/or in a dishwasher.

3. Fragrance-containing hollow body according to one of the previous claims, **characterized in that** the hollow body withstands 5 or more, preferably 10 or more standard drying cycles of a tumble dryer or 10 or more standard washing cycles of a dishwasher with structural integrity.

4. Fragrance-containing hollow body according to one of the previous claims, wherein as further substances one or more stabilizers are embedded in the polymer matrix in an amount in the range from 5 to 60 wt.-%, preferably in the range from 10 to 60 wt.-%, further preferably in the range from 20 to 55 wt.-%, particularly preferably in the range from 30 to 50 wt.-%, in each case with respect to the total weight of the hollow body.

5. Fragrance-containing hollow body according to claim 4, wherein the, one of, several of or all of the stabilizers are pourable at 25 °C and 1013 mbar and have a boiling point of higher or equal 180 °C at 1013 mbar, preferably have a boiling point of higher or equal 220 °C at 1013 mbar, preferably have a boiling point of higher or equal 250 °C at 1013 mbar and preferably are selected from the group consisting of phtalic acid esters, paraffines, polyalkylene glycols, polyalkylene glycol ethers or polyalkylene glycol esters,
and/or
are solid and selected from the group of clay minerals and inorganic pigments and preferably are selected from the group consisting of talcum, chalk, calcium sulfate, calcium carbonate, bentonite, zinc oxide, titanium oxide and montmorillonite.

6. Fragrance-containing hollow body according to one of the previous claims, **characterized in that** the total amount of the fragrances embedded into the elastomeric polymer matrix is in the range from 10 to 50 wt.-%, preferably in the range from 10 to 40 wt.-% and particular preferably in the range from 15 to 30 wt.-%, in each case with respect to the total weight of the hollow body.

7. Fragrance-containing hollow body according to one of the previous claims, **characterized in that** the hollow body still contains 40 wt.-% or more of the total amount of the fragrances originally present in the hollow body after 5 standard drying cycles of a tumble dryer or after 5 standard washing cycles of a dishwasher.

8. Fragrance-containing hollow body according to one of the previous claims, **characterized in that** in each standard tumble dryer or standard dishwasher cycle 50 mg or more of fragrances are released.

9. Fragrance-containing hollow body according to one of the previous claims, **characterized in that** the elastomeric polymer matrix is cold-cross-linked.

10. Fragrance-containing hollow body according to one of the previous claims, **characterized in that** the elastomeric polymer matrix is the reaction product
i) of a cross-linker from the group of epoxides, isocyanates, anhydrides or acrylates
with
ii) one, two or more polyamines
or comprises the same.

11. Fragrance-containing hollow body according to one of the previous claims, **characterized in that** the elastomeric polymer matrix is the reaction product
i) of 2-5 wt.-% of a cross-linker from the group of epoxides, isocyanates, anhydrides or acrylates, preferably 2-5 wt.-% of a cross-linker from the group of the isocyanates
with
ii) a total of 10 to 35 wt.-%, preferably 15 to 30 wt.-% of one, two or more polyamines
or comprises the same,
wherein the wt.-% values are in each case with respect to the total weight of the hollow body.

12. Fragrance-containing hollow body according to claim 10 or 11, **characterized in that** the, one of, both of or several of the polyamides are pourable, preferably liquid, at 25 °C and 1013 mbar.

13. Fragrance-containing hollow body according to one of the previous claims, **characterized in that** the volume of the cavity is in the range from 40 vol.-% to 98 vol.-%, preferably in the range from 70 vol.-% to 95 vol.-%, with respect to the total volume of the fragrance-containing hollow body at 25 °C and 1013 mbar.

14. Use of a fragrance-containing hollow body according to one of the claims 1 to 13 for applying a fragrance to laundry during a drying process in a tumble dryer and/or for the application of fragrance to dishwasher air during a washing process of a dishwasher, wherein the hollow body is suitable for multiple use.

15. Method for the production of a fragrance-containing hollow body according to one of the claims 1 to 13
comprising or consisting of the following steps:
a) providing one or more reagent(s) for the production of an elastomeric, preferably cross-linked polymer matrix,
b) addition of 10 to 75 wt.-% of one or more fragrance(s),
c) optionally addition of 5 to 60 wt.-%, preferably in a range from 10 to 60 wt.-%, preferred in a range from 20 to 55 wt.-%, further preferred in a range from 30 to 50 wt.-%, stabilizers as well as optionally further components,
d) mixing of reagents, fragrances, optionally stabilizers and optionally further components so that the elastomeric, preferably cross-linked, polymer matrix is formed,
e) forming of a hollow body, preferably by a spinning process using the mixture produced in step d),
wherein the wt.-% values are in each case with respect to the total weight of the hollow body.

## Revendications

1. Corps qui contient de la substance odoriférante et entoure de tous côtés un espace creux unique et qui comprend ou se compose de:
- une matrice polymère élastomère, de préférence réticulée et
- une ou plusieurs substances odoriférantes enrobées dans ladite matrice polymère, en une quantité totale comprise entre 10 et 75 % en poids, par rapport au poids total du corps creux,
- le cas échéant, d'autres substances enrobées dans ladite matrice polymère, dans lequel
- ledit corps creux présente un volume total compris entre 5 et 8181 cm³ à 25 °C et 1013 mbar,
et
- l'épaisseur de la paroi entourant de tous côtés ledit espace creux est comprise, sur chaque point, entre 0,25 et 20 mm, de préférence entre 0,5 et 15 mm, d'une manière particulièrement préférée entre 0,75 et 10 mm, le plus préférablement entre 1 et 8 mm.

2. Corps creux contenant de la substance odoriférante, selon la revendication 1, dans lequel le corps creux se prête à l'utilisation, de préférence à une utilisation multiple dans un sèche-linge et/ou dans un lave-vaisselle.

3. Corps creux contenant de la substance odoriférante, selon l'une quelconque des revendications précédentes, **caractérisé par le fait que** ledit corps creux résiste, avec une intégrité structurelle, à 5 cycles standard de séchage ou plus, de préférence à 10 cycles standard de séchage ou plus d'un sèche-linge ou à 10 cycles standard de rinçage ou plus d'un lave-vaisselle.

4. Corps creux contenant de la substance odoriférante, selon l'une quelconque des revendications précédentes, dans lequel un ou plusieurs agents stabilisants sont enrobés comme autres substances dans ladite matrice polymère élastomère, en une quantité comprise entre 5 et 60 % en poids, de préférence entre 10 et 60 % en poids, de préférence encore entre 20 et 55 % en poids, d'une manière particulièrement préférée entre 30 et 50 % en poids, respectivement par rapport au poids total du corps creux.

5. Corps creux contenant de la substance odoriférante, selon la revendication 4, dans lequel ledit, un, plusieurs ou tous les agent(s) stabilisant(s) sont coulables à 25 °C et 1013 mbar et présentent un point d'ébullition supérieur ou égal à 180 °C à 1013 mbar, de préférence un point d'ébullition supérieur ou égal à 220 °C à 1013 mbar, de manière préférée un point d'ébullition supérieur ou égal à 250 °C à 1013 mbar, et sont choisis de préférence dans le groupe comprenant les esters de l'acide phtalique, les paraffines, les polyalkylène glycols, les éthers de polyalkylène glycols ou les esters de polyalkylène glycols,
et/ou
sont solides et choisis dans le groupe comprenant les minéraux argileux et les pigments inorganiques et sont choisis de préférence dans le groupe comprenant le talc, la craie, le sulfate de calcium, le carbonate de calcium, la bentonite, l'oxyde de zinc, le dioxyde de titane et la montmorillonite.

6. Corps creux contenant de la substance odoriférante, selon l'une quelconque des revendications précédentes, **caractérisé par le fait que** la quantité totale des substances odoriférantes enrobées dans ladite matrice polymère élastomère est comprise entre 10 et 50 % en poids, de préférence entre 10 et 40 % en poids et d'une manière particulièrement préférée entre 15 et 30 % en poids, respectivement par rapport au poids total du corps creux.

7. Corps creux contenant de la substance odoriférante, selon l'une quelconque des revendications précédentes, **caractérisé par le fait que**, après 5 cycles standard de séchage d'un sèche-linge ou après 5 cycles standard de rinçage d'un lave-vaisselle, ledit corps creux contient encore 40 % en poids ou plus de la quantité totale des substances odoriférantes présentes à l'origine à l'intérieur du corps creux.

8. Corps creux contenant de la substance odoriférante, selon l'une quelconque des revendications précédentes, **caractérisé par le fait que** 50 mg ou plus de substances odoriférantes sont libérés respectivement par cycle standard de sèche-linge ou par cycle standard de lave-vaisselle.

9. Corps creux contenant de la substance odoriférante, selon l'une quelconque des revendications précédentes, **caractérisé par le fait que** la matrice polymère élastomère est réticulée à froid.

10. Corps creux contenant de la substance odoriférante, selon l'une quelconque des revendications précédentes, **caractérisé par le fait que** la matrice polymère élastomère est ou contient le produit de réaction
i) d'un réticulant provenant du groupe des époxydes, isocyanates, anhydrides ou acrylates
avec
ii) un, deux polyamine(s) ou plus.

11. Corps creux contenant de la substance odoriférante, selon l'une quelconque des revendications précédentes, **caractérisé par le fait que** la matrice polymère élastomère est ou contient le produit de réaction de
i) 2 à 5 % en poids d'un réticulant provenant du groupe des époxydes, isocyanates, anhydrides ou acrylates, de préférence de 2 à 5 % en poids d'un réticulant provenant du groupe des isocyanates,
avec,
ii) dans l'ensemble, 10 à 35 % en poids, de préférence 15 à 30 % en poids d'une, de deux polyamine(s) ou plus,
les données en % en poids étant rapportées chacune au poids total du corps creux.

12. Corps creux contenant de la substance odoriférante, selon la revendication 10 ou 11, **caractérisé par le fait que** ladite, une, les deux ou plusieurs des polyamines sont coulables, de préférence liquides, à 25 °C et 1013 mbar.

13. Corps creux contenant de la substance odoriférante, selon l'une quelconque des revendications précédentes, **caractérisé par le fait que** le volume de l'espace creux est compris entre 40 % volumétrique et 98 % volumétrique, de préférence entre 70 % volumétrique et 95 % volumétrique, par rapport au volume total du corps creux contenant de la substance odoriférante, à 25 °C et 1013 mbar.

14. Utilisation d'un corps creux contenant de la substance odoriférante, selon l'une quelconque des revendications 1 à 13, pour appliquer un parfum sur le linge durant un processus de séchage dans un sèche-linge et/ou pour parfumer l'air du lave-vaisselle durant un processus de rinçage d'un lave-vaisselle, ledit corps creux se prêtant à être utilisé à plusieurs reprises.

15. Procédé de fabrication d'un corps creux contenant de la substance odoriférante, selon l'une quelconque des revendications 1 à 13,
comprenant ou consistant en les étapes suivantes :
a) fournir un ou plusieurs éduits pour la réalisation d'une matrice polymère élastomère, de préférence réticulée,
b) ajouter entre 10 et 75 % en poids d'une ou de plusieurs substances odoriférantes,
c) ajouter, le cas échéant, de 5 à 60 % en poids, de préférence dans un intervalle compris entre 10 et 60 % en poids, de manière préférée dans un intervalle compris entre 20 et 55 % en poids, de préférence encore dans un intervalle compris entre 30 et 50 % en poids, d'agents stabilisants ainsi que, le cas échéant, d'autres composants,
d) mélanger lesdits éduits, substances odoriférantes, le cas échéant agents stabilisants et, le cas échéant, autres composants de sorte que la matrice polymère élastomère, de préférence réticulée, est formée,
e) mouler un corps creux, de préférence par un moulage par centrifugation en utilisant le mélange produit à l'étape d),
les données en % en poids étant rapportées chacune au poids total du corps creux.
